# EUROPEAN PATENT SPECIFICATION

(11) **EP 0 639 172 B1**
(45) Date of publication and mention of the grant of the patent: **28.10.1998**
(21) Application number: 93913620.6
(22) Date of filing: 05.05.1993
(51) Int. Cl.: C07C 45/46, C07C 45/54

(54) **PROCESS FOR THE PREPARATION OF POLYHYDROXYBENZOPHENONES**
VERFAHREN ZUR HERSTELLUNG VON POLYHYDROXYBENZOPHENONEN
PROCEDE DE PREPARATION DE POLYHYDROXYBENZOPHENONES

(30) Priority: 06.05.1992 EP 92201285
(43) Date of publication of application: 22.02.1995
(73) Proprietor: TECHNISCHE UNIVERSITEIT DELFT, 2628 BL Delft (NL)
(72) Inventor: HOEFNAGEL, Anthonius Johannes, NL-2628 BL Delft (NL); VAN BEKKUM, Herman, NL-2628 BL Delft (NL)
(74) Representative: Smulders, Theodorus A.H.J., Ir.
(86) International application number: NL9300095
(87) International publication number: WO9322268

(56) References cited:
- EP-A- 0 334 096
- FR-A- 2 667 063
- US-A- 1 995 402
- US-A- 3 354 221
- CHEMICAL ABSTRACTS, vol. 107, 1987, Columbus, Ohio, US; abstract no. 39399q, ABE, TAKU ET AL. 'Hydroxybenzophenones.' & JP,A,61 282 335 (HONSHU PAPER CO.) 12 December 1986

## Description

The invention relates to a process for the preparation of polyhydroxybenzophenones. More in particular, the invention relates to a process for the preparation of polyhydroxybenzophenones by treating benzoic acid or a derivative thereof with a polyhydroxybenzene in the presence of a large pore zeolite as a catalyst.

Polyhydroxybenzophenones are known as useful intermediates for many reactions. For example, 2,4-dihydroxybenzophenone is an intermediate in the preparation of 4-O-octyl-2-hydroxybenzophenone, useful as UV absorbent. Many methods of preparing these polyhydroxybenzenes are known.

According to British patent specification 947,686, polystyrene plastics are made stable to ultraviolet light by addition of certain benzophenone derivatives. According to this patent specification, benzophenone derivatives were produced by reacting o-toluic acid and resorcinol (1,3-benzenediol). However, HCl is used in this process, which leads to an inferior product.

Fries rearrangements [K.Fries and G.Finck, Chem.Ber., 41, 4271 (1908)] in which catalysts such as AlCl₃, ZnCl₂, HgCl₂, SnCl₄ and FeCl₃ are used, can also be useful in the preparation of polyhydroxybenzophenones. The preparation of 2,4-dihydroxybenzophenone can be acccomplished with a good yield by the metal chloride catalyzed reaction of resorcinol with benzotrichloride, leading to the production of hydrochloric acid (Chem.Abstr.87, 210102 (1977)).

Fries rearrangements in which catalysts such as polymeric perfluorinated sulfonic acid resin (Nafion-H) and ion-exchange resin (Amberlyst-15) are used, have also been reported. The Fries rearrangement of aryl benzoates in the presence of ion-exchange resin catalysts is known from, e.g. Chemical Abstracts 104, 109139u, where reacting aryl benzoates in the presence of Nafion-XR or Amberlire 200C, as a result of a Fries rearrangement leads to the preparation of the corresponding benzophenones.

In Chem. Abstr. 107, 39399q (1987) a process for the preparation of 2,3,4-trihydroxybenzophenone from benzoic acid and 1,2,3-trihydroxybenzene in the presence of Amberlyst-15 has been disclosed.

US-A-3,354,221 discloses a process for the preparation of mono-hydroxybenzophenones through a Fries rearrangement reaction using zeolitic catalysts.

US-A-1,995,402 is directed to the preparation of aryl-ketones and phenolesters. Examples 2 and 3 show processes for the preparation of mono-hydroxybenzophenones using hydrosilicates of alumina as a catalyst.

Chemical Abstracts 111, 7061q, discloses the preparation of polyhydroxybenzophenones by benzoylation of polyhydroxybenzenes, in the presence of a sulfonic acid. According to this process, Amberlyst-15 was used as a catalyst in the reaction of pyrogallol (1,2,3-benzenetriol) and p-hydroxybenzoic acid in the presence of toluene under azeotropical removal of water to give 2,3,4,4'-tetrahydroxybenzophenone.

A direct Fries reaction of resorcinol and benzoic acid is given in the following Figure:

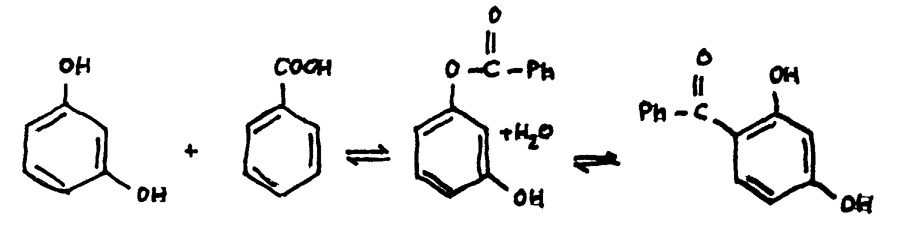

Water is the only by-product of the reaction, which can easily be removed.

The known processes to prepare benzophenone derivatives catalyzed by ion-exchange resins show some severe drawbacks, for instance, in the reaction of resorcinol with benzoic acid, where resorcinol dibenzoate is produced as a by-product and the resin is polluted with another, coloured, by-product. The formation of this coloured by-product, which is assumed to be 3,6-dioxy-9-phenyl-xanthydrol, decreases the yield of the desired compound and therefore lowers the efficiency of the process.

Amberlyst-15 is known to enhance the production of this coloured by-product, which renders the catalyst unusable after a period of time. Regeneration of such a catalyst is not possible as it will decompose at temperatures exceeding 200-250°C. Another drawback of the use of Amberlyst-15 is the fact that resorcinol is bound to the catalyst during the reaction, as a result of a sulfonation of this compound by the SO₃H groups of the catalyst. This also diminishes the efficiency of a regeneration of the catalyst.

The object of the present invention is to provide a process for the preparation of polyhydroxybenzophenones which does not have the drawbacks of the known processes.

The invention provides a method which allows polyhydroxybenzophenones to be produced with a good yield, with a minimized production of unwanted by-products.

Surprisingly, it has been found that using a large pore zeolite in the reaction of benzoic acid or a derivative thereof with a polyhydroxybenzene enables the reaction to be carried out in a one-pot operation, with a very good product yield, and without any, or with a strongly diminished, formation of unwanted by-products such as dibenzoates or any other by-products that decrease the efficiency of the catalyst.

The invention is therefore directed to a process for the preparation of polyhydroxybenzophenones by treating benzoic acid or a derivative thereof with a polyhydroxybenzene or a derivative thereof in the presence of a large pore zeolite as a catalyst. Preferably, the large pore zeolite catalyst is zeolite beta, and most preferred is zeolite H-beta, i.e., zeolite beta in the proton acid form.

In the method according to the invention the esterification reaction and Fries rearrangement reaction are catalyzed with one and the same catalyst in a one-pot operation, which simplifies the procedure and also diminishes the waste problem of the aforementioned known reactions with metal chlorides.

As all mother liquors, filtrates and washings that are used in the method according to the present invention can be recycled, and substantially no by-products are produced, the process is environmentally friendly whereas the known processes are not.

The use of a large pore zeolite, and more in particular of zeolite H-beta, does not lead to substantial production of the coloured by-product or dibenzoate esters. Additionally, the reaction mixture containing the end product can easily be separated from the catalyst by decantation, after which the product is isolated by extraction, for example with methanol.

A large pore zeolite is defined herein as a zeolite having pores with diameters equal to or larger than 0.6 nm, and preferably between 0.7 and 0.8 nm (Holderich, W.F. and van Bekkum, H., "Introduction to Zeolite Science and Practice", eds. van Bekkum, H., Flanigen, E.M. and Jansen, J.C., Elsevier, Amsterdam (1990), page 632).

The diameters of zeolites are obtained using X-ray techniques and absorption measurements. Newsam, J.M. et.al., Proc.R.Soc., London, A 240 (1988), page 375, describe the pore structure of zeolite beta in more detail.

The reaction can be carried out in conventional equipment suitable for this kind of chemical reactions.

The reaction suitably takes place by introducing the reactants, solvent, if any, and catalyst in the reactor with stirring under conditions of temperature and pressure suitable for the reaction to run. The reaction can be carried out continuously, discontinuously or semi-continuously.

Compounds which can be reacted using the method according to the present invention are polyhydroxybenzenes or derivatives thereof and benzoic acid or derivatives thereof.

Polyhydroxybenzenes can be substituted with one or more alkyl groups and/or halogen atoms. Resorcinol and derivatives thereof are preferred.

Benzoic acid can be substituted with one or more alkyl groups, like methyl or ethyl, and/or hydroxy atoms. o-Toluic acid and benzoic acid are preferred.

The reaction can be carried out in the presence or absence of a solvent. Preferably a solvent is used.

The solvent in which the reaction can be performed is preferably a high boiling, inert solvent, preferred boiling point at least 110°C, in which the starting materials, as well as the products, are soluble. The solvent does preferably not form a stable mixture with water.

Suitable solvents are, for example, n-butylbenzene (bp 182°C), p-chlorotoluene (bp 162°C), mesitylene (bp 165°C), n-decane (bp 174°C), and chlorobenzene (bp 130°C). Preferred solvents are n-butylbenzene and p-chlorotoluene.

If a solvent is present, the polyhydroxybenzene and derivatives thereof and benzoic acid and derivatives thereof are each present in an amount of at least 1 % by weight of the solvent, and the solubility in the solvent determines the maximum amount present.

In any case it is preferred that the reacting compounds are present in equimolar or substantially equimolar amounts, i.e., the molar ratio of benzoic acid to polyhydroxybenzene ranges from 0.5:1 to 2:1, preferably from 0.9:1 to 1.25:1.

If desired, the reaction can be performed under a nitrogen atmosphere.

The reaction temperature is preferably reflux temperature, and reaction times will be in the range of 10 minutes to 10 hours, more particularly in the range of 1 to 4 hours.

The pressure to be used is not critical. Atmospheric, sub-atmospheric and supra-atmospheric pressures may be used. In view of the ease thereof, atmospheric pressure is preferred.

A major amount of the reaction product can be obtained by simple decantation of the final reaction mixture and catalyst, and another part of the reaction product can be obtained by extraction of the catalyst with a suitable solvent, for example an alcohol like methanol, followed by a washing step with a suitable non-solvent like n-pentane and a drying step.

The filtrate (the solvent, if present) and catalyst can be reused after the reaction. The catalyst can be regenerated at elevated temperatures in the range of 250 to 450°C. The regeneration temperature is dependent on the type of catalyst. Zeolite H-beta can be regenerated at temperatures up to 400°C.

In a preferred embodiment of the invention, resorcinol is reacted with benzoic acid in n-butylbenzene in the presence of zeolite H-beta, resulting in 2,4-dihydroxybenzophenone.

In another preferred embodiment of the invention, 2-methylresorcinol is reacted with 2-methylbenzoic acid in p-chlorotoluene in the presence of zeolite H-beta, resulting in 2,4-dihydroxy-2',3-dimethylbenzophenone.

The invention will now be elucidated in and by the following examples, which are not intended to restrict the invention.

### Example 1

### Calcination of the zeolite catalyst

Zeolite beta (molar ratio Si/Al = 14, Exxon Chemical, Rotterdam) was calcined at 550°C for two days. After calcination, the zeolite was refluxed three times for one hour, with an 1 M ammonium nitrate solution, filtered off, washed with water, dried at 100°C for one hour and subsequently calcined at 550°C for two days.

Before use, the catalyst was heated to 400°C.

All other chemicals were commercial analytical grade products and were used without further purification.

### Preparation of 2,4-diOH-2',3-di-Me-benzophenone

1.55 g (12.5 mmol) 2-Me-resorcinol, 1.70 g (12.5 mmol) 2-Me-benzoic acid, 1.25 g zeolite H-beta (after activation at 400°C) and 50 ml n-decane were mixed. The mixture was stirred at reflux temperature (oil bath 190°C) with removal of condensed water (from a Dean-Stark condenser).

The course of the reaction was followed by GC-analysis (VARIAN, model 3700 GC, using a 50 M capillary column (sil 5 CP) at a temperature of 225°C).

After 3 h of refluxing, the yield of 2,4-diOH-2',3-di-Me-benzophenone was 90.7%.

After warm decantation of the clear light yellow solution, practically pure 2,4-diOH-2',3-di-Me-benzophenone precipitated. Filtration gave 1.6 g of a light yellow product.

A second portion (0.75 g) was obtained by extraction of the catalyst and reaction flask with methanol (40°C). After washing with n-pentane, the yield was 2.20 g product (98 % purity), i.e., 73 % of the theoretical maximum.

M.p 187-188.5°C.

¹H NMR analysis was in agreement with the proposed structure. The filtrate (n-decane) and the catalyst can be reused directly or after re-activation by heating for 2 hours at 400°C.

### Example 2

### Preparation of 2,4-diOH-2',3-di-Me-benzophenone

According to the method of Example 1, 2-Me-resorcinol and 2-Me-benzoic acid were reacted in the presence of zeolite H-beta (after activation at 400°C). 50 ml p-chlorotoluene was used as a solvent.

After 2 h of refluxing, the yield of 2,4-diOH-2',3-di-Me-benzophenone was 92 %.

After warm filtration - to remove the catalyst - the filtrate, after cooling, filtration, washing with n-pentane and drying, gave 2.25 g (i.e. 74 % of the theoretical value) 2,4-diOH-2',3-di-Me-benzophenone, with a purity of 97 % and a m.p. of 187-188.5°C.

### Example3

### Preparation of 2,4-diOH-2'-Me-benzophenone

According to the method of Example 1, 2.20 g (20 mmol) of resorcinol was reacted with 2.72 g (20 mmol) 2-Me-benzoic acid in 50 ml of mesitylene, in the presence of 2.00 g of zeolite H-beta (after activation at 400°C) as a catalyst.

After 3 h of refluxing with removal of condensed water from the condenser, the yield of 2,4-diOH-2'-Me-benzophenone was 90 %, according to GC-analysis.

Further processing according to the method of Example 1 yielded 3.48 g (i.e. 76 % of the theoretical value) of a practically pure product (98 % purity) with a melting point of 121-123°C.

The catalyst can be regenerated by heating at 400°C for 1 h after removal of the remainder of the reaction products and can be reused together with the filtrates.

### Example 4

### Preparation of 2,4-diOH-2'-Me-benzophenone

The method according to Example 3 was repeated with 50 ml of p-chlorotoluene as solvent.

Further processing according to the method of Example 1 yielded 3.47 g (i.e. 76 % of the theoretical value) of practically pure 2,4-diOH-2'-Me-benzophenone (98 % purity) with a melting point of 121-123°C.

### Example 5

### Preparation of 2,4-diOH-benzophenone and recycling of the catalyst and filtrate

A mixture of 1.10 g (10 mmol) of resorcinol, 1.22 g (10 mmol) of benzoic acid, 1.00 g zeolite H-beta (activated at 400°C) and 40 ml of n-butylbenzene was refluxed with stirring for 3 hours in a nitrogen atmosphere, resulting in an equilibrium mixture containing 11% of benzoic acid, 11% of resorcinol, 23% of resorcinol monobenzoate and 55% of 2,4-di-OH-benzophenone.

Isolation of 0.85 g (40 % of the theoretical value) of the light-yellow coloured product 2,4-diOH-benzophenone with a m.p. of 142-143.5°C was accomplished by warm filtration, followed by filtration of the cooled filtrate (20°C) and drying of the precipitate after washing with pentane and water.

### Recycling procedure

Recycling of the filtrate and the residues, obtained after evaporation of the extracts of the precipitate (extracted with pentane and water) and of the catalyst (extracted with methanol), in combination with the regenerated catalyst and with replenishment of 4 mmol of benzoic acid and of resorcinol, resulted in an equilibrium mixture containing 5% of benzoic acid, 5% of resorcinol, 24% of resorcinol monobenzoate and 66% of 2,4-dihydroxybenzophenone.

The reduced formation of water thus resulted in a more complete esterification reaction.

Isolation and purification according to the method of Example 1, produced 0.75 g (35%) of practically pure (98%) 2,4-di-OH-benzophenone with a m.p. of 144-145°C.

A conversion of the additionally added 4 mmol of benzoic acid and of resorcinol into 88% of practically pure 2,4-dihydroxybenzophenone has thus occurred.

### Comparative Examples

According to the method of Example 1, 10 mmol resorcinol was reacted with 10 mmol benzoic acid in 25 ml chlorobenzene, in the presence of various catalysts. The catalysts were present in an amount of 1 mmol or 1.0 g (zeolite or ion-exchange resin).

The yield of 2,4-diOH-benzophenone was determined and the production of by-products was measured.

The results are summarized in the following Table.

**TABLE**

| | | | | |
|---|---|---|---|---|
| Reaction mixtures obtained after refluxing of a solution of resorcinol and benzoic acid in chlorobenzene, using various catalysts. | | | | |

| Catalyst | time h % | resorcinol monobenzoate % | 2,4-diOH-benzo phenone % | resorcinol dibenzoate |
|---|---|---|---|---|
| H₃PO₄ | 24 | 55 | 9 | -** |
| CH₃SO₃H | 23 | 32 | 17 | - |
| Amberlyst 15 * | 6 | 16 | 67 | 4 |
| | 23 | 15 | 62 | 10 |
| Nafion 117 * | 6 | 24 | 46 | 2 |
| | 24 | 23 | 45 | 3 |
| Zeolite H-beta | 4 | 16 | 10 | - |
| | 24 | 17 | 20 | trace |

| | | | | |
|---|---|---|---|---|
| * Ion-exchange resins were intensively coloured after reaction. | | | | |
| ** not detectable. | | | | |

The results of the Table clearly show that the use of ion-exchangers such as Amberlyst-15 or Nafion 117 as a catalyst, leads to the production of resorcinol dibenzoate as a by-product.

Further, the ion-exchangers were polluted with a coloured by-product, which is assumed to be 3,6-dioxy-9-phenylxanthydrol. This by-product decreases the yield of the desired compound and therefore diminishes the efficiency of the process. Also, resorcinol is sulfonated and bound to the ion-exchangers, which also diminishes the efficiency of the reaction.

In using zeolite beta as a catalyst, hardly any resorcinol dibenzoate is produced and the catalyst is not intensively coloured. Zeolite beta, unlike ion-exchangers, is easily regenerated at high temperatures, for example at 250 to 400°C, to remove any organic material and water.

## Claims

1. A process for the preparation of polyhydroxybenzophenones by reacting benzoic acid or a derivative thereof with a polyhydroxybenzene or a derivative thereof in the presence of a zeolite having pores with diameters equal to or larger than 0,6 nm as a catalyst.

2. A process according to claim 1, wherein the derivative of benzoic acid is an alkyl substituted benzoic acid.

3. A process according to claim 2, wherein the alkyl substituted benzoic acid is o-toluic acid.

4. A process according to claim 1 or 2, wherein the derivative of benzoic acid is a halogen substituted benzoic acid.

5. A process according to claim 1, wherein the polyhydroxybenzene or a derivative thereof is resorcinol.

6. A process according to claims 1 to 5, wehrein the reaction is performed in a solvent, preferably in n-butylbenzene or p-chlorotoluene.

7. A process according to claim 6, wherein the reaction is performed by mixing the benzoic acid or derivative thereof and the polyhydroxybenzene or a derivative thereof with a solvent in the presence of a large pore zeolite.

8. A process according to claims 1 to 7, wherein the reaction is performed by stirring the reaction mixture at reflux temperature.

9. A process according to claims 1 to 8, wherein during the reaction water is removed from the reaction mixture.

10. A process according to claims 1 to 9, wherein the large pore zeolite is zeolite beta.

11. A process according to claims 1 to 10, wherein the zeolite is used in the proton acid form.

12. A process according to claims 1 to 11, wherein the said zeolite is regenerated before use.

## Patentansprüche

1. Verfahren zur Herstellung von Polyhydroxybenzophenonen durch Umsetzen von Benzoesäure oder einem Derivat hiervon mit einem Polyhydroxybenzol oder einem Derivat hiervon in Gegenwart eines Zeolithen mit Poren mit Durchmessern gleich oder größer 0,6 nm als Katalysator.

2. Verfahren Anspruch 1, wobei das Derivat der Benzoesäure eine alkylsubstituierte Benzoesäure ist.

3. Verfahren nach Anspruch 2, wobei die alkylsubstituierte Benzoesäure o-Toluylsäure ist.

4. Verfahren nach Anspruch 1 oder 2, wobei das Derivat der Benzoesäure eine halogensubstituierte Benzoesäure ist.

5. Verfahren nach Anspruch 1, wobei das Polyhydroxybenzol oder ein Derivat hiervon Resorcinol ist.

6. Verfahren nach einem der Ansprüche 1 bis 5, wobei die Reaktion in einem Lösungsmittel durchgeführt wird, bevorzugt in n-Butylbenzol oder p-Chlortoluol.

7. Verfahren nach Anspruch 6, wobei die Reaktion durch Vermischen der Benzoesäure oder einem Derivat hiervon und des Polyhydroxybenzols oder einem Derivat hiervon mit einem Lösungsmittel in Gegenwart eines Zeolithen mit großen Poren durchgeführt wird.

8. Verfahren nach einem der Ansprüche 1 bis 7, wobei die Reaktion durch Verrühren der Reaktionsmischung bei Refluxtemperatur durchgeführt wird.

9. Verfahren nach einem der Ansprüche 1 bis 8, wobei während der Reaktion aus der Reaktionsmischung Wasser abgezogen wird.

10. Verfahren nach einem der Ansprüche 1 bis 9, wobei der großporige Zeolith Beta-Zeolith ist.

11. Verfahren nach einem der Ansprüche 1 bis 10, wobei der Zeolith in der Protonensäureform eingesetzt wird.

12. Verfahren nach einem der Ansprüche 1 bis 11, wobei der Zeolith vor der Anwendung regeneriert wird.

## Revendications

1. Un procédé de préparation de polyhydroxybenzophénones par réaction de l'acide benzoïque ou d'un de ses dérivés avec un polyhydroxybenzène ou un de ses dérivés, en présente d'une zéolite ayant des pores d'un diamètre égal ou supérieur à 0,6 nm en tant que catalyseur.

2. Un procédé selon la revendication 1, dans lequel le dérivé de l'acide benzoïque est un acide benzoïque alkylé.

3. Un procédé selon la revendication 2, dans lequel l'acide benzoïque alkylé est l'acide o-toluique.

4. Un procédé selon la revendication 1 ou 2, dans lequel le dérivé d'acide benzoïque est un acide benzoïque halogéné.

5. Un procédé selon la revendication 1, dans lequel le polyhydoxybenzène ou son dérivé est le résorcinol.

6. Un procédé selon la revendication 1, dans lequel on effectue la réaction dans un solvant, dc préférence le n-butylbenzène ou le p-chlorotoluène.

7. Un procédé selon la revendication 6, dans lequel on effectue la réaction par mélange de l'acide benzoïque ou de son dérivé et du polyhydoxybenzène ou de son dérivé, avec un solvant en présence d'une zéolite à grand pore.

8. Un procédé selon les revendications 1 à 7, dans lequel on effectue la réaction par agitation du mélange réactionnel à la température du reflux.

9. Un procédé selon lcs revendications 1 à 8, dans lequel. au cours de la réaction, on élimine l'eau du mélange réactionnel.

10. Un procédé selon les revendications 1 ou 9, dans lequel la zéolite à grand pore est la zéolite béta.

11. Un procédé selon les revendications 1 ou 10, dans lequel la zéolite est utilisée sous forme acide protoné.

12. Un procédé selon les revendication 1 ou 11, dans lequel ladite zéolite est régénérée avant emploi.
